# EUROPEAN PATENT APPLICATION

(11) **EP 1 291 437 A1**
(43) Date of publication of application: **12.03.2003**
(21) Application number: 01120717.2
(22) Date of filing: 05.09.2001
(51) Int. Cl.: C12Q 1/25

(54) **New assay for inhibitors of translocases**

(71) Applicant: BAYER AG, 51368 Leverkusen (DE)
(72) Inventor: Hewlett, Guy, Dr., 42327 Wuppertal (DE)

(57) **Abstract**

The present invention is directed to a method for the detection of inhibitors of nucleic-acid dependent translocases in homogeneous phase comprising the displacement of a biotin labelled oligonucleotide from its complex with avidin or streptavidin in the presence of a biotinylated dye.

## Description

The present invention is directed to a method for the detection of inhibitors of nucleic-acid dependent translocases in homogeneous phase comprising the displacement of a biotin labelled oligonucleotide from its complex with avidin or streptavidin in the presence of a biotinylated dye.

Successful application of High Throughput Screening (HTS) technology is crucial for modem drug discovery as the quantity of compounds to be tested until a pharmaceutically active compound is found is steadily rising.

This requires the transformation of standard laboratory techniques into automated processes, hence raising the need to eliminate or circumvent steps like multiple washings, decanting, or chromatographic separations, all of which cannot be easily automated. Use of radioactively labelled compounds should also be avoided in order to prevent machinery being contaminated.

A further requirement for success in HTS is that assay designs deliver a signal to noise ratio big enough to avoid large amounts of data being falsely deemed positive. This makes it necessary to embed a detection step which ideally is a direct measure for the specific interaction between a test-compound and its target and will not be induced by non-specific interactions.

Contemplating the above, the objective of the current invention is to develop an homogeneous, high throughput microplate assay for prokaryote and eukaryote nucleic acid translocases such as polymerases and, in particular, helicases.

Helicases are nucleic acid-dependent translocating enzymes that unwind duplex nucleic acids to provide single stranded substrates for subsequent nucleic acid-modifying or replicating activities. During the course of unwinding, helicases also translocate along the nucleic acid whereby both processes are coupled to the hydrolysis of nucleoside triphosphates. Helicases are found in prokaryotic and eukaryotic organisms and are also encoded by viruses. As such they represent viable targets for therapeutic intervention in pathogenic infections and cancer.

Early assays for estimating helicase activity were not suitable for HTS and either depended on the correlation of NTPase activity with helicase translocation or used radioactively labelled, partially duplex substrates, the unwinding of which can be detected by gel electrophoresis. More modem assay formats have used the sensitivity of duplex DNA to single strand-specific nucleases, fluorescence energy transfer, displacement of fluorescently labelled oligonucleotides from pre-formed duplexes, displacement of duplex-specific fluorescent dyes and scintillation proximity assays.

Alaoui-Ismaili et al. (2000; Antiviral Research 46: 181-193 ) e.g. describe an automated screening assay for HCV NS3 helicase activity using a cumbersome radioactive scintillation proximity assay in 96-well format which suffers from a poor signal to noise ratio.

Sivaraja et al. describe a screening assay for helicase enzymes (1998; Analytical Biochemistry 265: 22-27), however this assay is in ELISA format which requires multiple washing steps and makes use of radioactively labelled compounds.

Eggleston et al. (1996; Nucleic Acids Research 24: 1179-1186) describe a helicase assay based on the displacement of fluorescent, nucleic acid-binding ligands by measuring the release of intercalating fluorescent dye from double stranded nucleic acids as a result of helicase activity. However the reported signal to noise ratio between bound and released dyestuff is insufficient for an effective statistical analysis of experimental data for helicase inhibitors in a high throughput screening setting.

In conclusion assays as known from literature so far are slow, expensive and cumbersome and do not lend themselves easily to high throughput screening since the ideal HTS assay should feature homogeneity, low price, precision and the activity should be demonstrated by a positive signal.

Considering all the above, it can be summarised that until now an assay suited for the assessment of helicase activity on an industrial scale using HTS is not available.

The current invention now comprises a novel method of an homogeneous assay for the high throughput screening for inhibitors of helicases and related nucleic acid translocating enzymes, which can be achieved when combining the ability of helicases to displace biotin-labelled oligonucleotides from their complex with avidin or streptavidin and the ability of dyes like biotin-labelled fluorescein to bind to the free avidin or streptavidin binding sites for their detection.

As known DNA helicases are able to displace biotin-labeled oligonucleotides from their complex with streptavidin (Morris and Raney (1999), Biochemistry 38: 5164-5171), but this displacement was demonstrated on an experimental scale only in a classical gel-based assay rather than in solution, and for detection of that displacement movement of a radioactively-labelled oligonucleotide was measured which moved faster when compared to its complex with streptavidin or avidin.

As is further known, binding sites of avidin or streptavidin for biotin can be quantitated in crude biofluids when applying certain biotin-fluorescein conjugates (Kada, G. et al. (1999), Biochimica et Biophysica Acta 1427: 33-43).

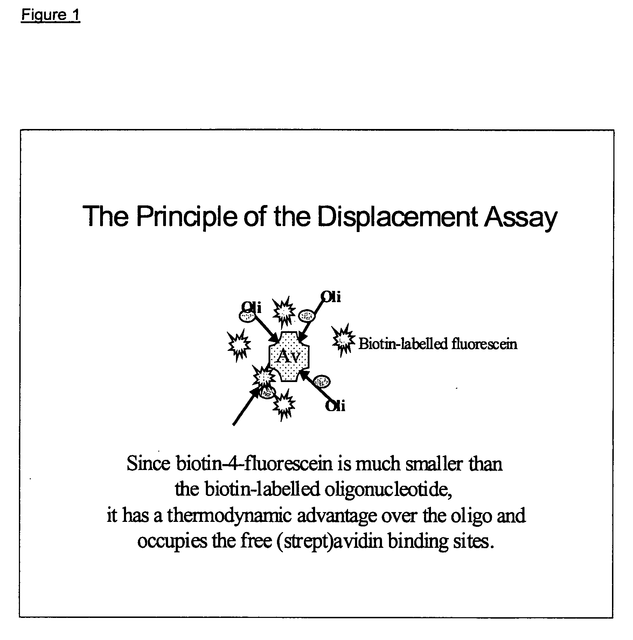

The transformation of that knowledge now leads to an approach for the design of an assay which is run in homogeneous phase and delivers an excellent signal/noise ratio. It can be successfully applied for the discovery of inhibitors of helicases on an industrial scale where a biotin-labelled oligonucleotide is displaced from the (strept)avidin binding site and so permits the binding of the small biotin-labelled fluorescein to the free site. Figure 1

### Experimental

### Example 1

### A Helicase Assay with single-stranded DNA (ssDNA)

The helicase substrate takes the form of an oligonucleotide of length appropriate to the helicase under test that is biotinylated at one end, appropriate to the directionality of the helicase under test. In this example, a single-strand 60-mer with the sequence GACGTATTCAAGATACCTCGTACTCTGTACTGACTGCGATCCGACTGTCC TGCATGATG was labelled with biotin at the 5'-terminus (obtained from Eurogentec, Belgium) so that it could be used as a substrate for the helicase function of the SV40 large T antigen which is a 3'-5' helicase.

Equal volumes of streptavidin and biotin-labelled oligonucleotide dissolved in reaction buffer (see below) were mixed in such a way that there was at least a four-fold molar excess of oligonucleotide over streptavidin. This is to ensure the saturation of all biotin-binding sites on the streptavidin molecule (4 biotin-binding sites per molecule streptavidin). This mixture was incubated for at least 15 minutes before being used for the assay.

An aliquot of the oligonucleotide/streptavidin complex (oligo/SA) was then mixed with SV40 large T antigen and biotin-4-fluorescein in a suitable buffer, in this case 50mM Tris-HCl pH 7.5, containing 3mM MgCl₂, 5mM dithiothreitol and 100 µg/ml bovine serum albumin and pipetted into 384-well microplates.

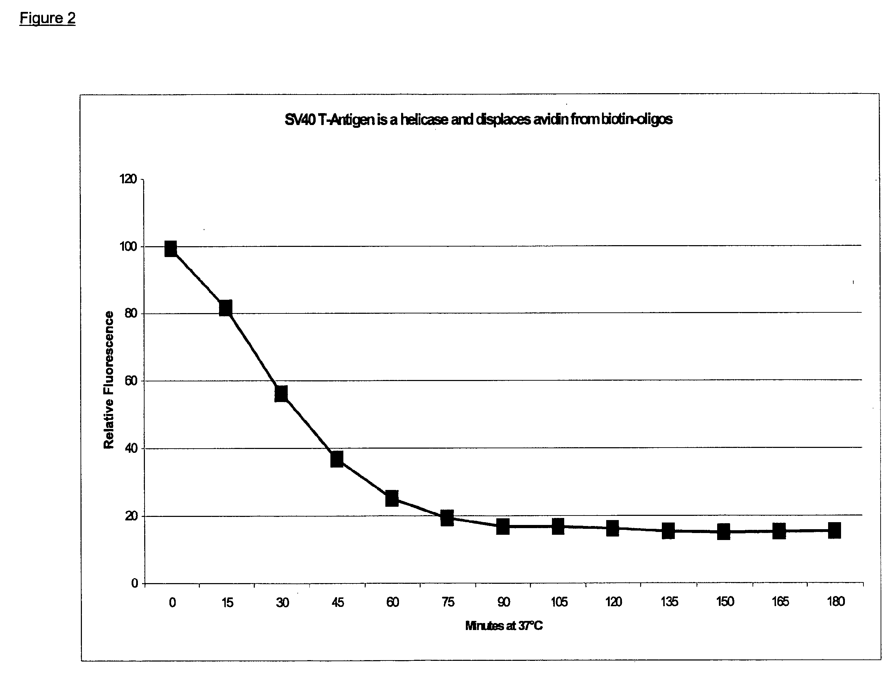

The enzyme reaction was started by adding an appropriate amount of adenosine triphosphate and incubating the plate at 37°C. Measurements were made at an excitation wavelength of 485nm and an emission wavelength of 535nm using a microplate fluorometer. The data of a typical reaction with SV40 large T-antigen are summarised in Figure 2 and show helicase activity as demonstrated by the loss of signal caused by the quenching of the biotin-fluorescein when it binds to free streptavidin binding sites.

### Example 2

### A Helicase Assay with double-stranded DNA (dsDNA)

The helicase substrate takes the form of an oligonucleotide of length appropriate to the helicase under test that is biotinylated at one end, appropriate to the directionality of the helicase under test. In this example, a single-strand 60-mer with the sequence GACGTATTCAAGATACCTCGTACTCTGTACTGACTGCGATCCGACTGTCC TGCATGATG was labelled with biotin at the 5'-terminus (obtained from Eurogentec, Belgium). This was then annealed to a complementary strand with the sequence TCTGTACTGACTGGTATCTTGAATACGT (Eurogentec, Belgium) so that it could be used as a double-stranded substrate for the helicase function of the SV40 large T antigen which is a 3'-5' helicase.

Equal volumes of streptavidin and biotin-labelled oligonucleotide duplex dissolved in reaction buffer (see below) were mixed in such a way that there was at least a four-fold molar excess of oligonucleotide over streptavidin. This is to ensure the saturation of all biotin-binding sites on the streptavidin molecule (4 biotin-binding sites per molecule streptavidin). This mixture was incubated for at least 15 minutes before being used for the assay.

An aliquot of the duplex/streptavidin complex (oligo/SA) was then mixed with SV40 large T antigen and biotin-4-fluorescein in a suitable buffer, in this case 50mM Tris-HCl pH 7.5, containing 3mM MgCl₂, 5mM dithiothreitol and 100 µg/ml bovine serum albumin and pipetted into 384-well microplates.

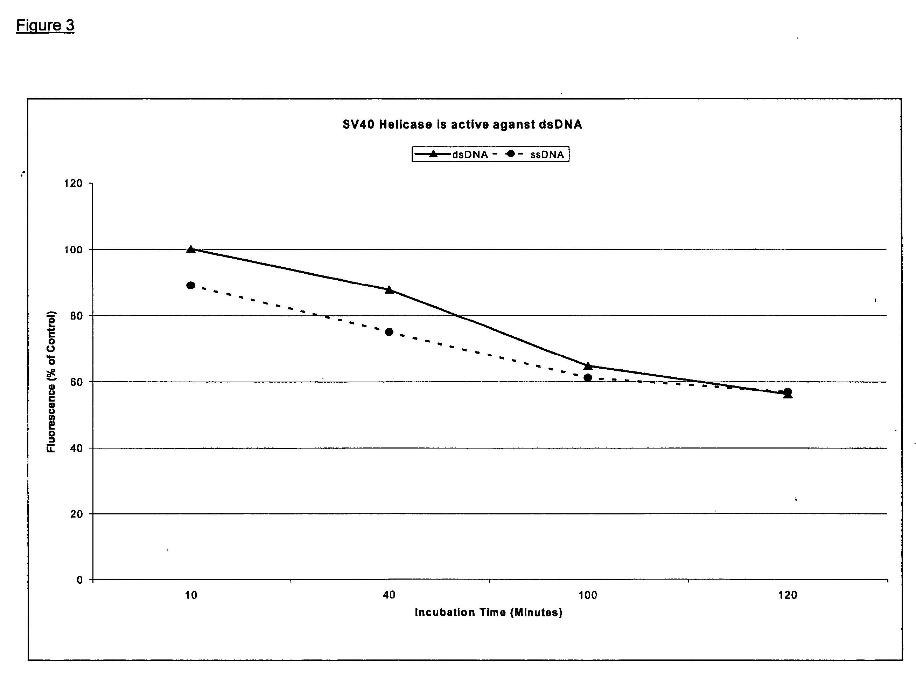

The enzyme reaction was started by adding an appropriate amount of adenosine triphosphate and incubating the plate at 37°C. Measurements were made at an excitation wavelength of 485nm and an emission wavelength of 535nm using a microplate fluorometer. The data of a typical reaction with SV40 large T-antigen are summarised in figure 3 and demonstrate the loss of signal caused by the quenching of the biotin-fluorescein when it binds to streptavidin binding sites made available by the action of the helicase activity of SV40 large T-antigen on the double-stranded DNA substrate.

### Example 3

### Characterisation of known helicase inhibitors using the helicase assay

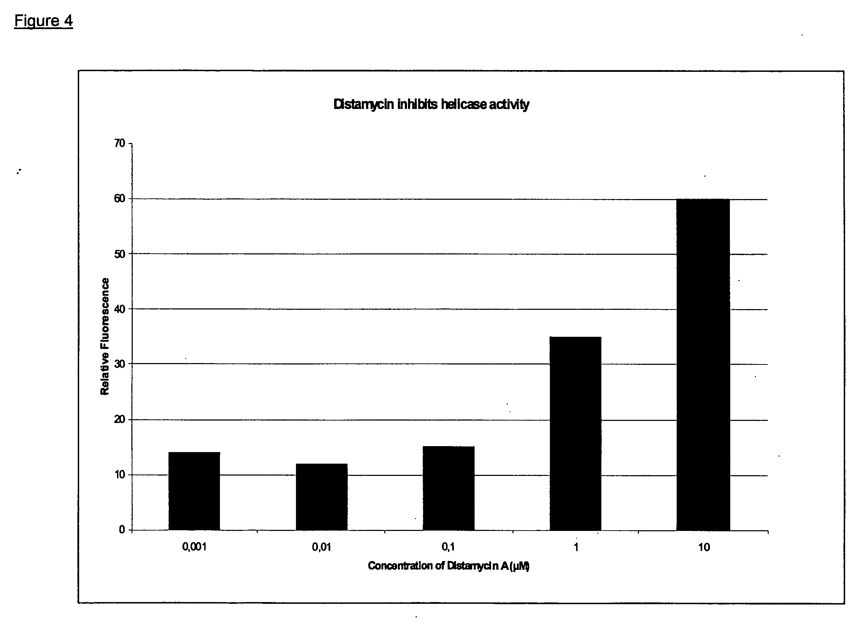

A known inhibitor of helicase activity, Distamycin A, which is a DNA minor groove binder (Soderlind, K.J. et al. (1999; Anti-Cancer Drug Design 14: 19-36) was added to the helicase assay as described above and was found to dose-dependently decrease helicase activity, i.e. inhibit displacement of the streptavidin from the biotin-labelled substrate and so increase the signal at the time of measurement (figure 4).

## Claims

1. Method for the detection of inhibitors of nucleic-acid dependent translocases in homogeneous phase comprising the displacement of a biotin labelled oligonucleotide from its complex with avidin or streptavidin in the presence of a biotinylated dye

2. Method according to 1 where the nucleic-acid dependent translocase is a helicase

3. Method according to 1 and 2 wherein the biotin-labelled oligonucleotide is a single- or double-stranded or partially double-stranded DNA or RNA

4. Method according to 1 to 3 wherein the biotin unit is either attached to a 5' or a 3' end of the oligonucleotide.

5. Method according 1 to 4 wherein the biotinylated dye is a biotinylated fluorescein

6. Use of a method according 1 to 5 in an automated or High Throughput Screening Setting

7. Use of a method according 1 to 6 for the discovery of inhibitors of translocases and helicases

8. Use of inhibitors of translocases and helicases found with methods according 1 to 7 for the production of pharmaceutical compositions


SEQUENCE LISTING

<110>  Bayer AG

<120>  New Assay for inhibitors of translocases

<130>  LeA 35410

<160>  2

<170>  PatentIn version 3.1

<210>  1
<211>  59
<212>  DNA
<213>  Artificial

<400>  1
gacgtattca agatacctcg tactctgtac tgactgcgat ccgactgtcc tgcatgatg      59

<210>  2
<211>  28
<212>  DNA
<213>  Artificial

<400>  2
tctgtactga ctggtatctt gaatacgt                                        28